(19) Europäisches Patentamt
European Patent Office
Office européen des brevets

(11) Veröffentlichungsnummer: **0 476 549 B1**

# (12) EUROPÄISCHE PATENTSCHRIFT

(45) Veröffentlichungstag der Patentschrift: **16.08.95**

(51) Int. Cl.6: **C12Q 1/32**, C12Q 1/37, C12N 1/00, C12P 13/20

(21) Anmeldenummer: **91115613.1**

(22) Anmeldetag: **14.09.91**

## (54) Verfahren und Anordnung zur enzymatischen Bestimmung von Aspartam und dafür geeigneter Zellextrakt.

(30) Priorität: **17.09.90 DE 4029296**

(43) Veröffentlichungstag der Anmeldung: **25.03.92 Patentblatt 92/13**

(45) Bekanntmachung des Hinweises auf die Patenterteilung: **16.08.95 Patentblatt 95/33**

(84) Benannte Vertragsstaaten: **CH DE FR LI NL**

(56) Entgegenhaltungen:

**BIOSENSORS & BIOELECTRONICS, Band 6,1991, Elsevier Science Publishers Ltd. (GB); K.B. MALE et al., Seiten 117-123/**

**ANALYTICA CHIMICA ACTA, Band 234, 1990, Amsterdam (NL); A. MULCHANDANI et al., Seiten 465-469/**

**ANALYTICA CHIMICA ACTA, Band 245, 1991, Amsterdam (NL); M.T. JEPPESEN et al., Seiten 89-99/**

**ANALYTICA CHIMICA ACTA, Band 206, 1988, Amsterdam (NL); G.G. GUILBAULT et al., Seiten 369-374/**

(73) Patentinhaber: **FORSCHUNGSZENTRUM JÜLICH GMBH**
**Wilhelm-Johnen-Strasse**
**D-52425 Jülich (DE)**

(72) Erfinder: **Hummel, Werner, Dr.**
**Claudiusstrasse 11**
**W-5177 Titz (DE)**

**EP 0 476 549 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren und eine Anordnung zur Bestimmung des Gehalts an L-α-Aspartyl-L-phenylalanin-methylester (Asp-PheOMe; Aspartam®) in wäßriger Lösung durch enzymatische Spaltung in Asparaginsäure und Phenylalaninmethylester mittels Peptidase und nachfolgende enzymatische Nachweisreaktion, und sie umfaßt einen dafür geeigneten enzymatisch-aktiven Zellextrakt.

Der Süßstoff Aspartam ist ein Dipeptidester aus den beiden L-Aminosäuren Asparaginsäure und Phenylalanin, der wegen seiner hohen Süßkraft und physiologischen Unbedenklichkeit innerhalb kurzer Zeit in zahlreichen Ländern zugelassen worden ist und mittlerweile in einer Größenordnung von mehreren 1.000 to/Jahr hergestellt wird.

Wichtige Einsatzgebiete für diesen Süßstoff sind z.B. Erfrischungsgetränke, Süßstofftabletten und Streusüße, Dessert, Fruchtjoghurt und Kaugummi.

Aspartam ist nur in fester Form bei Raumtemperatur völlig stabil, in wäßrigen Lösungen hängt die Stabilität von der Zeit, der Temperatur und dem pH-Wert ab. Abbauprodukte der Hydrolyse können Aspartylphenylalanin, Phenylalaninmethylester, Asparaginsäure und Phenylalanin sein, Es besteht daher ein erhebliches Interesse an einer möglichst selektiven leicht zu praktizierenden Aspartam-Analytik.

Deren bedeutsame Anwendungsbereiche sind einerseits die Prozeßkontrolle bei der Herstellung dieses Dipeptids, andererseits die Qualitätskontrolle der Endprodukte (Lebensmittel), besonders unter dem Aspekt der eingeschränkten Lagerstabilität von Aspartam. So bleibt beispielsweise der Geschmack von Erfrischungsgetränken nur erhalten, wenn eine Lagertemperatur von 20-25 °C eingehalten wird und ein Verzehr innerhalb von ca. 5 Wochen gewährleistet ist. Ein weiteres Anwendungsgebiet für eine Aspartam-Analytik liegt im medizinischen Bereich, wo in biologischen Proben, beispielsweise Serum oder Urin, Abbau- und Ausscheidungsraten bestimmt werden müssen.

Bekanntermaßen wird die Aspartam-Analyse in aller Regel mittels HPLC durchgeführt (z.B. Stamp, J.A., Labuza, T.P., Journal of Food Science 54, (1989), 1043-1046). Daneben sind Bestimmungsmethoden mittels Dünnschicht-Chromatographie, Kapillar-Isotachophorese, Gaschromatographie oder Ionenaustauscher-Chromatographie (Aminosäure-Analysator) beschrieben. Diese Verfahren erfordern einen erheblichen Aufwand und sind im allgemeinen für hohe Probensequenzen weniger geeignet.

Es gibt auch bereits Aspartam-Analysenverfahren, die auf einer enzymatischen Umsetzung basieren: So wird von O. Fatibello-Filho u.a. in Anal.Chem. 60 (1988) Seiten 2397-99 ein Aspartam-Analysenverfahren beschrieben, das auf einer enzymatischen Umsetzung desselben zunächst mit Carboxypeptidase A unter Abspaltung von L-Asparaginsäure und nachfolgende Umsetzung mit L-Aspartase zu Fumarat und Ammoniak basiert. Hierbei werden die Enzyme auf der Gasmembran einer Elektrode coimmobilisiert, die auf die gebildeten Ammoniumionen reagiert.

Ein ähnliches Verfahren der gleichen Arbeitsgruppe wird von G.G. Guilbault u.a. in Anal.Chem.Acta 206 (1988) Seiten 369-74 beschrieben, bei dem ebenfalls eine ammoniumselektive Elektrode verwendet wird, auf der L-Aspartase immobilisiert ist, mit deren Hilfe ein unmittelbarer Umsatz von Aspartam unter Abspaltung von Ammoniak erfolgt. Daneben werden von den Autoren unterschiedliche enzymatische Abbauwege skizziert, die zum Teil über mehrere Stufen verlaufen und im allgemeinen unter Ammoniakentwicklung ablaufen sollen, die jedoch nicht näher untersucht wurden.

Aus Anal.Chim.Acta 234 (1990) Seiten 465-69 ist schließlich ein Aspartam-Analysenverfahren bekannt, bei dem mittels Peptidase Asparaginsäure abgespalten wird, die in Gegenwart von α-Ketoglutarat mittels Apartat-Aminotransferase zu L-Glutamat umgesetzt wird, dessen enzymatische Oxidation mittels Glutamat-Oxidase unter Bestimmung des Sauerstoffverbrauchs der Gehaltbestimmung zugrundegelegt wird.

Ein Problem dieser enzymtischen Analysentechniken besteht in der relativ geringen Stabilität der L-Aspartase und darin, daß die erzielbaren Aussagen nicht unbedingt eindeutig sind.

Ziel der Erfindung ist daher ein Aspartam-Analysenverfahren, das mit relativ stabilen gut verfügbaren Enzympräparaten durchgeführt werden kann, glatt abläuft und verläßliche Aussagen ermöglicht:
Das zu diesem Zweck entwickelte erfindungsgemäße Verfahren der eingangs genannten Art ist im wesentlichen dadurch gekennzeichnet, daß man als Nachweisreaktion eine adenindinukleotid-cokatalysierte enzymatische Reaktion herbeiführt, indem man entweder

(a) die gebildete Asparaginsäure mittels eines Asparaginsäure in Gegenwart von $NADP^+$ umsetzenden enzymhaltigen Zellextrakts unter Zugabe von $NADP^+$ umsetzt und die Konzentration des Aspartams über die NADPH- oder $NH_3$-Bildung ermittelt oder

(b) die Estergruppe vom Phenylalaninmethylester enzymatisch mittels Chymotrypsin abtrennt und das gebildete L-Phenylalanin enzymatisch mittels Phenylalanin-Dehydrogenase in Gegenwart von $NAD^+$ in Phenylpyruvat umwandelt und die Aspartam-Konzentration über die NADH- oder $NH_3$-Bildung ermittelt.

Bei dieser Verfahrensweise wird für die Bestimmung jeweils von enzymatischen Umsetzungen Gebrauch gemacht, die unter Nicotinamiddinukleotid-Coenzymzusatz ablaufen unter Bildung der Coenzymhydrierungsprodukte, die besonders zweckmäßig auf physikalischem Wege, insbesonder fluorimetrisch nachweisbar sind.

Dabei wird entweder das L-Phenylalanin des Aspartams mit Phenylalanin-Dehydrogenase (Phenylalanin-DH) für die Konzentrationsbestimmung von Aspartam nach dem folgenden Prinzip herangezogen:

Peptidase

1. Asp-PheOMe - - - - -> Aspartat + Phenylalanin-methylester

CHYMOTRYPSIN

2. Phenylalanin-methylester - - - - - - - -> Phenylalanin + Methanol

PHENYLALANIN-DH

3. Phenylalanin + $NAD^+$ - - - - - - - - -> Phenylpyruvat + $NH_4^+$ + NADH

Eine brauchbare Phenylalanin-DH ist in der EP-PS 188 712 beschrieben.

Oder die Gehaltsbestimmung erfolgt mittels eines neuen enzymatisch wirkenden Zellextraktes, der Asparaginsäure in Gegenwart von $NADP^+$ unter Bildung von NADPH und Ammoniak umsetzt, gemäß folgendem Prinzip:

Peptidase

1. Asp-PheOMe - - - - -> Aspartat + Phenylalaninmethylester

Zellextrakt

2. Aspartat + $NADP^+$ - - - - - -> Asp-Rk.-prod. + NADPH + $NH_4^+$

Beide Varianten können besonders vorteilhaft als Fließinjektionsanalyse (FIA) durchgeführt werden, für die in den beigefügten Figuren 1 und 2 Fließschemen angedeutet sind. Die Enzyme werden dabei in immobilisierter Form in aufeinanderfolgenden Säulen eingesetzt, durch die ein im allgemeinen durch Pufferlösung gebildeter Trägerstrom geschickt wird, in den sowohl die Probe als auch eine bestimmte Konzentration des jeweiligen Coenzyms injiziert werden. Das anläßlich der enzymatischen Reaktion 2 bzw. 3 gebildete NADPH bzw. NADH wird insb. fluorimetrisch bestimmt und dient als Maß für die Aspartamkonzentration der Probe.

Für die Peptidspaltung stehen dem Fachmann unterschiedliche Peptidasen zur Verfügung. Im Rahmen der vorliegenden Untersuchungen wurde vorwiegend Pronase® verwendet.

Die Aspartambestimmung mittels Phenylalanin-DH läuft mithin folgender Maßen ab:

1. Die Aspartam-haltige Probe passiert die peptidase-haltige Enzymsäule, wobei Aspartam zu Asparaginsäure und Phenylalaninmethylester gespalten wird.
2. Die Probe passiert die chymotrypsin-haltige zweite Säule, wobei der entstanden Phenylalaninmethylester in Phenylalanin und Methanol gespalten wird.
3. Die Probe passiert die dritte Säule, die das Enzym Phenylalanin-Dehydrogenase enthält. Hier wird Phenylalanin oxidativ desaminiert unter Reduktion des Coenzyms. Das entstandene reduzierte Coenzym NADH kann als Maß für die Aspartam-Konzentration quantifiziert werden (z.B. durch Messung der Fluoreszenz), obwohl selbstverständlich auch die Ammoniumbildung herangezogen werden kann.

An der Stelle der fluorimetrischen NADH-Bestimmung kann jedes andere physikalische Nachweisverfahren wie z.B. eine photometrische oder amperometrische Bestimmung vorgesehen werden.

Hintergrundreaktionen, die nicht auf Aspartam basieren, können relativ einfach bestimmt werden, indem ein weiterer Aliquot der Probe derart injiziert wird, daß die Probe nur die Chymotrypsin- und die Phenylalanin-Dehydrogenase-Säule passiert. Damit werden das bereits vor der Analyse vorliegende Phenylalanin bzw. dessen Ester quantitativ erfaßt und können zur Korrektur herangezogen werden.

Für die Analytik sollten die drei Enzyme möglichst räumlich voneinander getrennt wie z.B. an unterschiedlichen Stellen auf einer fixierenden Oberfläche (z.B. Membran) verwendet werden, da zwei der eingesetzten Enzyme (Pronase bzw. Peptidase und Chymotrypsin) Proteasen sind, die sowohl die Phenylalanin-Dehydrogenase als auch sich gegenseitig inaktivieren würden. Besonders zweckmäßig ist hier die FIA-Technik mit Anwendung von gesonderten Enzym-Säulen, die nacheinander passiert werden.

Für die Bestimmung des Aspartamgehalts gemäß o.g. Variante (a) mittels einer cokatalysierten Asparaginsäureumwandlung wurde ein enzymatisch aktiver Zellextrakt gefunden, der als Rohextrakt verwendbar ist und Asparaginsäure NADP-abhängig unter Bildung von $NH_3$ und NADPH umsetzt.

Bei dieser Analysenvariante tritt also an die Stelle der oben genannten Schritte 2 und 3 eine Umsetzung der mittels Peptidase aufgespaltenen Probe durch besagten Zellextrakt - in FIA-Technik eine Passage lediglich durch eine weitere Säule mit träger-fixiertem enzymatisch wirksamen Zellextrakt. (Für die Berücksichtigung von Hintergrundreaktionen und die räumliche Trennung der beteiligten Enzyme gilt das o.g. analog).

Die Gewinnung des Rohextrakts erfolgt aus Mikroorganismen, die mittels eines Screeningverfahrens aus Bodenproben, insbesondere aus Kompostierungsproben, gewonnen wurden.

Geeignete Stämme konnten aus unterschiedlichen Bodenproben nach dem weiter unten in Beispiel 5 beschriebenen Verfahren isoliert werden, wie z.B. aus Proben von Laubwald-Oberflächneboden, Haus-(misch)kompost und kommunalen Kompostierungsanlagen.

Näher untersucht wurde dabei ein als Asp-1 bezeichneter Organismus, der unter der Nr. DSM 6705 bei der deutschen Sammlung von Mikroorganismen in Braunschweig hinterlegt worden ist.

Der aus diesen Organismen erzielte Rohextrakt ist selbstverständlich nicht nur für die Aspartambestimmung geeignet, sondern generell als Katalysator für die enzymatische Asparaginsäurebestimmung nützlich.

Nachfolgend wird die Erfindung im einzelnen anhand von Beispielen unter Bezugnahme auf die angefügten Zeichnungen beschrieben. Es zeigen im einzelnen:

| | |
|---|---|
| Figur 1 und 2 | Anordnungen zur Fließinjektionsanalyse von Aspartam |
| Figur 3 und 5 | Kurvenbilder für die Detektorsignale abhängig von der Aspartam-Konzentration bei der Aspartamanalyse durch FIA-Technik mit Phenylalanin-DH (Fig. 3) bzw. Asparaginsäure-aktivem Zellextrakt (Fig. 5) und |
| Figur 4 | eine Kurve für die NADPH-Bildungsrate in Abhängigkeit von der L-Asparaginsäure-Konzentration. |

Beispiel 1

Spaltung von Apartam mittels Peptidase

Für die Untersuchung der Peptidspaltung wurden 10 mM Aspartam, gelöst in 100 mM Tris-HCl-Puffer, pH 7,5 (mit 10 mM $CaCl_2$) mit 2 Einheiten einer Peptidase versetzt und bei 30 °C inkubiert. Nach 10 bis 20 min wurden Proben (je 50 µl) entnommen, wobei die enzymatische Spaltungsreaktion durch Zusatz von 200 µl Säure (pH 2,2) abgestoppt wurde. Das durch proteolytische Wirkung aus Aspartam freigesetzte Aspartat wurde mit Hilfe eines Aminosäure-Analysators quantitativ bestimmt und diente als Maß für die Spaltungsaktivität.

Von den untersuchten Peptidasen zeigten die Pronase® der Firma Boehringer Mannheim eine besonders hohe Aktivität und zwar wurden mit Pronase® 214 nMol freigesetztes Aspartat/min x ml erzielt.

Das als Pronase® bekannte Enzym ist eine Mischung von Peptidasen, Esterasen und Proteasen, die das im Gemisch wirksame aspartamspezifische Peptidaseenzym enthält.

Beispiel 2: Immobilisierung von Pronase, Chymotrypsin und Phenylalanin-Dehydrogenase

Für die Immobilisierung können beispielsweise kommerziell erhältliche poröse Feststoffe eingesetzt werden, die bereits in geeigneter Weise für die Kopplung von Enzymen vorbehandelt (aktiviert) sind. Im einzelnen wurden eingesetzt:

A. Immobilisierung von Pronase:

53 mg $SiO_2$-Träger*(Porengröße 540 Å; Fa. Mobitec, Göttingen, FRG) werden in 400 µl Kaliumphosphat-Puffer (pH 7,0) suspendiert, dazu werden 100 µl Pronase-Lösung (17,5 mg/350 µl $H_2O$ = 122,5 U/350 µl

* Mobitec-Träger aktiviert durch Isocyanatgruppen

$H_2O$) zugesetzt. Diese Suspension wird 30 min bei 4° C leicht geschüttelt, danach für 20 Std. bei 4°C ruhend aufbewahrt. Dann wird über einer Fritte mit 500 ml Tris-HCl-Puffer (0,1 M; pH 7,0) gewaschen, dieser Puffer wird auch zum Aufbewahren des Immobilisats verwendet. Die enzymatische Aktivität dieses Immobilisats wird durch folgenden Test bestimmt:
500 µl einer 10 mM Aspartam-Lösung (gelöst in 100 mM Tris-HCl-Puffer, pH 7,5 (mit 10 mM $CaCl_2$)) werden mit 33 mg Pronase-haltigem Immobilisat versetzt und bei 30° C inkubiert. Nach 10 und 20 min werden Proben (je 50 µl) entnommen, wobei die enzymatische Spaltungsreaktion durch Zusatz von 200 µl Säure (pH 2,2) abgestoppt wird. Das durch proteolytische Wirkung aus Aspartam freigesetzte Aspartat wird mit Hilfe eines Aminosäure-Analysators quantitativ bestimmt und dient als Maß für die Spaltungsaktivität (1 Enzymeinheit (1 U) ist die Enzymmenge, die 1 µMol Aspartat/min bildet).
Die Immobilisierung unter diesen Bedingungen liefert einen Träger mit 7,7 U Pronase pro 1 g Träger.

B. Immobilisierung von Chymotrypsin:

51 mg $SiO_2$-Träger (Porengröße 540 Å; Fa. Mobitec, Göttingen, FRG) werden in 400 µl Kaliumphosphat-Puffer (pH 7,0) suspendiert, dazu werden 50 µl Chymotrypsin-Lösung (4 mg (360 U)/ml $H_2O$) zugesetzt. Diese Suspension wird 30 min bei 4° C leicht geschüttelt, danach für 20 Std. bei 4° C ruhend aufbewahrt. Dann wird über einer Fritte mit 500 ml Tris-HCl-Puffer (0,1 M; pH 7,0) gewaschen, dieser Puffer wird auch zum Aufbewahren des Immobilisats verwendet. Die enzymatische Aktivität dieses Immobilisats wird durch folgenden Test bestimmt:
500 µl einer 10 mM Lösung von L-Phenylalanin-Methylester (gelöst in 100 mM Tris-HCl-Puffer, pH 7,5 (mit 10 mM $CaCl_2$)) werden mit 34 mg Pronase-haltigem Immobilisat versetzt und bei 30° C inkubiert. Nach 10 und 20 min werden Proben (je 50 µl) entnommen, wobei die enzymatische Spaltungsreaktion durch Zusatz von 200 µl Säure (pH 2,2) abgestoppt wird. Das durch enzymatische Wirkung aus Aspartam freigesetzte L-Phenylalanin wird mit Hilfe eines Aminosäure-Analysators quantitativ bestimmt und dient als Maß für die Spaltungsaktivität (1 Enzymeinheit (1 U) ist die Enzymmenge, die 1 µMol Phenylalanin/min bildet).
Die Immobilisierung unter diesen Bedingungen liefert einen Träger mit 4,88 U Chymotrypsin pro 1 g Träger.

C: Immobilisierung von Phenylalanin-Dehydrogenase:

55 mg $SiO_2$-Träger (Porengröße 540 Å; Fa. Mobitec, Göttingen, FRG) werden in 400 µl Kaliumphosphat-Puffer (pH 7,0) suspendiert, dazu werden 100 µl Phenylalanin-Dehydrogenase-Lösung (170 U/ml) zugesetzt. Diese Suspension wird 30 min bei 4° C leicht geschüttelt, danach für 20 Std. bei 4° C ruhend aufbewahrt. Dann wird über einer Fritte mit 500 ml Tris-HCl-Puffer (0,1 M; pH 7,0) gewaschen. Die gebundene Aktivität wird mit folgendem Test gemessen: 20 mg immobilisiertes Enzym, 400 µl Ammoniumformiat-Puffer (0.1 M; pH 8,3), 10 µl Formiat-Dehydrogenase (50 U/ml) 50 µl Phenylpyruvat (50 mM) und 25 µl NADH (10 mM). Nach 10 und 20 min werden Proben entnommen, durch Zusatz von Säure abgestoppt und das durch enzymatische Reaktion jeweils gebildete Phenylalanin mit Hilfe eines Aminosäure-Analysators (Biotronik 6001, München, BRD; Trennprogramm für Proteinhydrolysate) gemessen. (1 Enzymeinheit (1 U) ist die Enzymmenge, die 1 µMol Phenylalanin/min bildet). Man erhält damit Träger mit einem Enzymgehalt von 6-8 U/g Träger.

Beispiel 3

Aufbau eines Analysensystems zur Aspartem-Bestimmung und Eichung

Der schematische Aufbau des Analysensystems (Prinzip der Fließ-Injektions-Analyse) ist aus Fig. 1 ersichtlich. $P_1$, $P_2$ und $P_3$ sind SchlauchPumpen; zur simultanen Injektion von Probe und Coenzym in den Pufferstrom wird ein Doppel-Injektionsventil der Fa. Tecator (40 µl) eingesetzt. Von den enzymhaltigen Säulen E enthielt $E_1$: Pronase, $E_2$: Chymotrypsin und $E_3$: Phenylalanin-Dehydrogenase; F ist ein Fluoreszenz-Photometer.

Das $NAD^+$ kann selbstverständlich auch an anderer Stelle zugegeben werden, sofern es nur für die Dehydrogenase-Reaktion zur Verfügung steht.

Die NADH-Konzentration wird mit Hilfe eines Fluoreszenz-Detektors (Merck F-1050; Merck; Darmstadt, FRG) mit der Anregungswellenlänge 340 nm und der Absorptionswellenlänge 460 nm gemessen. Die immobilisierten Enzyme sind in Säulen aus Plexiglas gefüllt worden (15 mm Länge, 3 mm Innendurchmesser), wobei das Austreten des Immobilisats durch feinmaschige Kunststoffnetze verhindert wird.

Die Säulen und der Detektor werden kontinuierlich von einem Pufferstrom (0,05 M Tris-HCl, pH 8,5 mit 10 mM $CaCl_2$) mit einem Fluß von 1,1 ml/min durchströmt. Die Probe wird mit Hilfe des Injektonsventils pulsartig in den Pufferstrom injiziert und passiert aufgrund des konstanten Flusses mit reproduzierbarer Verweilzeit die drei Enzymsäulen.

Aspartem-Konzentrationen im Bereich von 0,1 - 5 mM, gelöst in Natriumacetat-Essigsäure-Puffer (0,2 M, pH 4,3), ergeben einen linearen Zusammenhang zwischen der Aspartam-Konzentration und dem Fluoreszenzsignal (Fig. 3).

Die gesamte Meßzeit beträgt mit diesem Aufbau 95 sec., die Probenfrequenz liegt bei mind. 30 Proben/Std..

Beispiel 4: Anwendung der Analysenmethode auf Lebensmittel-Proben

Als Beispiel für die Anwendbarkeit der enzymatischen Bestimmungsmethode wurde der Aspartam-Gehalt einer Süßstoff-Tablette und zweier mit Aspartam gesüßten Lebensmittel (Erdbeer-Joghurt und Pfirsich-Quark, Fa. Onken) bestimmt. Zur Kontrolle wurde die Aspartam-Konzentration mit Hilfe eines Aminosäure-Analysators gemessen. Für die enzymatische Aspartam-Bestimmung wurden die Proben folgendermaßen vorbereitet: Eine Süßstoff-Tablette wurde in 20 ml Natriumacetat-Essigsäure-Puffer (0,2 M; pH 3,5) gelöst, 2 ml dieser Lösung wurden dann für die weitere Probenvorbereitung verwendet. Von den beiden Lebensmittelproben wurden jeweils 1,5 g mit 0,5 ml dieses Acetat-Puffers verrührt. Diese drei Proben wurden mit 10 ml Propylencarbonat versetzt, für 5 min im Ultraschallbad behandelt und dann 3 min bei 5000 rpm zentrifugiert. Die Propylencarbonat-haltige Phase wurde abgenommen und durch Zusatz von 2 g $Na_2SO_4$ (wasserfrei) getrocknet. Nach 2 Std. wurde der Feststoff über ein Papierfilter abfiltriert, 1:10 mit Natriumacetat-Essigsäure-Puffer (0,2 M; pH 4,3) verdünnt und der Aspartam-Gehalt mit Hilfe der in Beispiel 3 beschriebenen Fließ-Injektions-Analyse gemessen. In Tab. 2 sind diese Ergebnisse zusammengestellt. Für die Messung mit dem Aminosäure-Analysator wurden die Proben mit Natriumacetat-Essigsäure-Puffer (0,2 M; pH 2,2) aufgenommen, zur Entfernung von Fest- und Trübstoffen zentrifugiert und filtriert (0,45 µm-Filter) und am Aminosäure-Analysator (Biotronik 6001, München, BRD) mit dem Trennprogramm für Proteinhydrolysate aufgetrennt. Die Elutionszeit für Aspartam liegt unter diesen Bedingungen bei 49,3 min, mit Hilfe eingewogener Aspartam-Standards kann diese Trennmethode geeicht werden. Die Ergebnisse sind in Tab. 2 denen aus der FIA gegenübergestellt. Der Vergleich zeigt, daß die enzymatische Aspartam-Bestimmung für Lebensmittelproben anwendbar ist.

Tab. 2: Aspartam-Gehalte in Lebensmittelproben. Jede Probe wurde enzymatisch und mit Hilfe eines Aminosäure-Analysators gemessen.

| Probe | Aspartam-Gehalt (g/l) enzymatisch (FIA) | Aspartam-Gehalt (g/l) chromatographisch (Aminos.-Analysator) |
|---|---|---|
| Süßstoff-Tablette | 2,1 | 2,3 |
| Joghurt | 1,9 | 2,0 |
| Quark | 2,0 | 2,1 |

Für die Trägerfixierung wurden folgende Vergleichsexperimente durchgeführt:

A. PRONASE

A.1. Immobilisierung an Aminopropyl-CPG (CPG = Controlled Pore Glass), Porengröße 1400 Å (Fluka, Neu-Ulm, FRG): Immobilisierung entsprechend der Vorschrift von Stolzenbach, F.E. und Kaplan, N.O.

(1976) in Methods in Enzymology (Colowick, S.P. and Kaplan, N.O., eds.), Vol 44, pp 929-936, Academic Press, New York.
Kopplung entsprechend der Vorschrift mit Glutardialdehyd. Eingesetzt wurden 100 µl Pronase-Lösung (122,5 U/350 µl $H_2O$).
A.2. Wie A.1., aber Kopplung mittels CMC ( = 1-Cyclohexyl-3-(2-morpholinoethyl)carbodiimid (5 mg/ml).
A.3. Wie A.1., aber Kopplung mittels EDAC ( = 1-Ethyl-3-(3-dimethylaminopropyl)carbodiimid
A.4. Wie A.1., aber an Stelle von Aminopropyl-CPG wurde BIORAN (Fa. Schott) mit einer Porengröße von 550 Å eingesetzt.
A.5. Wie A.4., aber die Glutardialdehyd-Lösung enthielt zusätzlich noch 50 mg/ml Natriumborhydrid.

B. CHYMOTRYPSIN

B.1. Wie A.2., aber mit 50 µl Chmyotrypsin-Lösung (360 U/ml) statt Pronase.
B.2. Wie A.3., aber mit 50 µl Chmyotrypsin-Lösung (360 U/ml).

C. PHENYLALANIN-DEHYDROGENASE

C.1. Wie A.1., aber mit 100 µl Phenylalanin-Dehydrogenase (170U/ml) statt Pronase.
C.2. Wie A.2., aber mit 100 µl Phenylalanin-Dehydrogenase (170U/ml).
C.3. Wie A.3., aber mit 100 µl Phenylalanin-Dehydrogenase (170U/ml).

Tab.: Zusammenfassung der Ergebnisse zur Immobilisierung von Pronase, Chymotrypsin und Phenylalanin-Dehydrogenase.

| | gebundenes Enzym (U/g Träger) | | |
|---|---|---|---|
| Methode | Pronase | Chymotrypsin | Phenylalanin-DH |
| Beispiel 2.A. | 7,7 | | |
| A.1. | 3,9 | | |
| A.2. | 2,2 | | |
| A.3. | 2,4 | | |
| A.4. | 4,5 | | |
| A.5. | 3,3 | | |
| Beispiel 2.B. | | 4,88 | |
| B.1. | | 0,73 | |
| B.2. | | 0,38 | |
| Beispiel 2.C. | | | 8 |
| C.1. | | | 2 |
| C.2. | | | 6,5 |
| C.3. | | | 4 |

Wie man sieht, ist der mit reaktiven Isocyanatgruppen aktivierte $SiO_2$-Träger der Fa. Mobitec besonders geeignet.

Beispiel 5: Isolierung von Mikroorganismen mit asparaginsäure-aktivem Enzym

A. Isolierung aus Bodenproben:

Geeignete Mikroorganismen wurden aus Bodenproben isoliert, indem Aufschlämmungen von Bodenproben auf durch Agar-Zusatz verfestigtes Nährmedium aufgetragen wurden.

Das Nährmedium war folgendermaßen zusammengesetzt:

| | |
|---|---|
| L-Asparaginsäure | 10 g/l |
| Hefeextrakt | 0,1g/l |
| $KH_2PO_4$ | 2 g/l |
| Spurensalz-Lösung | 20 ml/l |
| Agar | 18 g/l |

Der pH-Wert wurde auf 5,5 eingestellt, die Lösung wurde 20 min bei 121 °C sterilisiert und dann in sterile Petrischalen abgefüllt.

Als Quelle für die Mikroorganismen dienten Bodenproben, die mit steriler Kochsalz-Lösung (9 g/L) aufgeschlämmt wurden, dazu wurde ca. 1 g Bodenprobe mit 20 ml Kochsalz-Lösung versetzt und ca. 20 min unter leichtem Rühren bei Raumtemperatur gehalten. Von dieser Suspension wurden Verdünnungsreihen angefertigt und diese auf dem verfestigten Nährmedium ausplattiert. Die Platten wurden bei 30 °C bebrütet, nach zwei Tagen wurden dann Einzelkolonien unter sterilen Bedingungen abgenommen und vereinzelt. Kolonien wurden als rein angesehen, wenn sowohl Verdünnungsausstriche auf Platten als auch das mikroskopische Bild einheitlich waren.

Reine Kolonien wurden in Flüssigmedium (obiges Medium ohne Agar; jeweils 100 ml) im Schüttelkolben (500 ml Erlenmeyer-Flaschen) überimpft und auf der Rundschüttelmaschine bei 120 UpM und einer Bebrütungstemperatur von 30 °C für 2 Tage inkubiert. Die Zellen wurden durch Zentrifugation geerntet, mit Puffer (0,1 M Kaliumphosphat; pH 7,5) gewaschen und schließlich mit Puffer (2 ml Puffer pro 1 g Bakterien-Feuchtmasse) suspendiert. In dieser Suspension wurden die Zellen durch Vermahlen mit Glasperlen (0,3 mm Durchmesser) aufgeschlossen. Durch Zentrifugation wurde ein klarer zellfreier Überstand (Rohextrakt) erhalten, der für den Enzymtest eingesetzt wurde. Die Isolate wurden daraufhin getestet, ob sie in der Lage sind, $NADP^+$-abhängig Asparaginsäure umzusetzen.

| Enzymtest: | |
|---|---|
| L-Asparaginsäure | 10 mM |
| Kaliumphosphat-Puffer | 100 mM (pH 8,5) |
| $NADP^+$ | 1 mM |
| $MgCl_2$ | 1 mM |
| Rohextrakt | 5-20 µl/ml (entspr. ca. 100 µg Protein/ml Test) |

Der komplette Ansatz ohne $NADP^+$ wurde kurzzeitig bei 30 °C vorinkubiert, dann wurde die Reaktion durch Zusatz von $NADP^+$ gestartet. Die Bildung von NADPH wurde bei 340 nm am Photometer verfolgt, Meßtemperatur war 30 °C. Für die Bestimmung der Enzymaktivität wurde mit Hilfe des Extinktionskoeffizienten für NADPH die maximale NADPH-Bildungsrate ermittelt. Eine mögliche Hintergrund-Aktivität wurde durch Bestimmung im Test ohne Asparaginsäure ermittelt. Als Enzymeinheit (1 Unit) wurde diejenige Enzymmenge definiert, die 1 µMol NADPH/1 min umsetzt.

Es wurden insgesamt ca. 40 Bodenproben eingesetzt, daraus wurden mehr als 200 Isolate erhalten, wobei aus mehreren Proben einer Kompostanlage Mikroorganismen mit der gewünschten Aktivität isoliert werden konnten. Auf Grund des makroskopischen Bildes handelte es sich in allen Fällen um denselben Organismus, beispielhaft wurde das Isolat "Asp-1" mikrobiologisch identifiziert und für die folgenden Anwendungen eingesetzt.

Taxonomische Bestimmung des Isolats Asp-1:

Die mikrobiologische Charakterisierung ergab, daß der Stamm Asp-1 der Gattung Pseudomonas zuzuordnen ist. Im einzelnen wurden folgende Eigenschaften festgestellt: Gram-negative Stäbchen, beweglich, strikt aerob, Oxidasepositiv, Katalase-positiv, Säurebildung aus Glucose, Abbau von Mannose, keine

Stärke-Hydrolyse, nichtfluoreszierend.

Der Stamm wurde bei der Deutschen Sammlung von Mikroorganismen , Braunschweig, unter der Nummer DSM 6705 hinterlegt.

Beispiel 6 : Charakterisierung des Enzymkomplexes

A. Nachweis der enzymabhängigen NADPH-Bildung

Um die Kopplung des Asparaginsäure-Abbaus und der Reduktion des Coenzyms NADP zu zeigen, wurde folgende Reaktionslösung in eine Küvette gefüllt (angegeben sind die Endkonzentrationen):

Tris-HCl-Puffer (0,125 M; pH 8,5;)
L-Asparaginsäure (1,86 mM)
$NADP^+$ (1 mM)
$MgCl_2$ (1 mM)
Rohextrakt Stamm Asp-1 (50 µl/ml)

Reaktionstemperatur ist 30 °C.

Die Bildung von NADPH wurde photometrisch bei 340 nm verfolgt, im Abstand von 1 min wurde eine Probe zur Bestimmung der aktuellen Asparaginsäure-Konzentration entnommen, gleichzeitig wurde die der NADPH-Bildung entsprechende Zunahme der Absorption bei 340 nM gemessen. Die Konzentration der Asparaginsäure wurde säulenchromatographisch mit Hilfe eines Aminosäure-Analysators (Biotronik LC 5001) bestimmt, die NADPH-Konzentration durch Umrechnung mit Hilfe des Absorptionskoeffizienten für NADPH. Tab. 1 zeigt, daß die Bildung von NADPH gekoppelt ist mit dem Abbau von Asparaginsäure.

Tab.1

| Enzymkatalysierte Bildung von NADPH und Abbau von Asparaginsäure | |
|---|---|
| abgebaute Asparaginsäure [µM] | gebildetes NADPH [µM] |
| 0 | 0 |
| 29 | 28 |
| 57 | 58 |
| 145 | 148 |
| 181 | 177 |

B. Nachweis der enzymgekoppelten $NH_3$-Bildung

Beim Abbau der Asparaginsäure wurde stöchiometrisch $NH_3$ freigesetzt. Zum Nachweis wurden aus einem kompletten Reaktionsansatz zu verschiedenen Zeiten Proben entnommen und die Konzentration der verbliebenen Asparaginsäure bzw. des gebildeten $NH_3$ mit Hilfe des Aminosäure-Analysators bestimmt. Um einen möglichst vollständigen Ablauf der Reaktion zu erreichen, wurde die Reaktion mit einer NADPH-verbrauchenden Reaktion gekoppelt. In diesem Fall wurde die enzymkatalysierte Reduktion von Acetophenon zum Phenylethanol verwendet; von dieser Reaktion ist bekannt, daß sie strikt NADPH-abhängig ist. Die Gewinnung dieses Enzyms wurde wie in der Literatur beschrieben durchgeführt (Hummel, W., Appl. Microbiol. Biotechnol. (1990) 34, 15-19), es wurde ein Präparat mit einer spezifischen Aktivität von 30 U/mg eingesetzt.

Der komplette Reaktionsansatz (1 ml Endvolumen) enthielt im einzelnen:

Tris-HCl-Puffer (0,1 M; pH 9,0)
L-Asparaginsäure (10 mM)
$NADP^+$ (1 mM)
Acetophenon (10 mM)
$MgCl_2$ (1 mM)
Phenylethanol-Dehydrogenase (5 U/ml)
Rohextrakt vom Stamm Asp-1 (100 µl/ml)

Nach einer Reaktionszeit von 20 min sind 3,4 µMol/ml Asparaginsäure abgebaut und 3,5 µMol/ml $NH_3$ gebildet worden, nach 60 min sind 6,6 µMol/ml Asparaginsäure abgebaut und 6,5 µMol/ml an $NH_3$ gebildet

worden.

C. Substratspezifität der enzymatischen Reaktion

Glutaminsäure wurde als strukturanaloge Verbindung als Substrat getestet um auszuschließen, daß es sich bei dem beschriebenen Enzym um eine Glutamat-Dehydrogenase handeln könnte. In einem Test wie in Beispiel 2.A beschrieben wurde L-Asparaginsäure durch L-Glutaminsäure ersetzt und die Reaktion photometrisch (340 nm) verfolgt. Innerhalb von 5 min wurde keine Bildung von NADPH gemessen. Dies zeigt, daß der Enzymkomplex aus dem Stamm Asp-1 spezifisch für L-Asparaginsäure ist.

Beispiel 7 : Verwendung des Enzymkomplexes für die Bestimmung der L-Asparaginsäure-Konzentration

Der Enzymkomplex aus dem Stamm Asp-1 kann für die Bestimmung von Asparaginsäure-Konzentrationen verwendet werden. Wie in den Beispielen 2.A und 2.B erläutert ist, wird aus Asparaginsäure durch die Wirkung des Enzyms in stöchiometrischem Verhältnis NADPH und auch $NH_3$ gebildet. Für die Asparaginsäure-Analytik können daher sowohl die Konzentration von freigesetztem $NH_3$ als auch diejenige von NADPH gemessen werden. Für die Messung von $NH_3$ bestehen eine Reihe von Analysenmethoden, beispielsweise mit Hilfe einer $NH_3$-sensitiven Elektrode, Gas-Analysatoren oder auch der Glutamat-Dehydrogenase. Wir haben beispielhaft die NADPH-Bildungsrate photometrisch gemessen. Dazu wurden im Testansatz nach Beispiel 2.A diverse Asparaginsäure-Konzentrationen eingesetzt und die Bildungsrate von NADPH (Zunahme von NADPH/min) in Abhängigkeit der diversen Asparaginsäure-Konzentrationen bestimmt. Wie Fig. 4 verdeutlicht besteht im Bereich von 0-2 mM ein direkter Zusammenhang zwischen der eingesetzten Asparaginsäure-Konzentration und der gemessenen NADPH-Bildungsrate.

Beispiel 8

Aspartam-Bestimmung mittels enzymatischer NADP-abhängiger Asparaginsäure-Bestimmung

Von den nach Aspartam-Spaltung mittels Pronase gebildeten Produkten wurde wahlweise Asparaginsäure zur Gehaltsbestimmung herangezogen: Der aus dem Stamm DSM 6705 (Asp-1) isolierte Enzymkomplex wurde auf einer Säule E4 (Fig. 2) immobilisiert und mittels des Detektors photometrisch das gebildete NADPH gemessen.

Dazu wurde der Enzymkomplex in der unter Beispiel 2 beschriebenen Weise an einen Träger immobilisiert. Wie in Beispiel 3 beschrieben, wurde dieses Immobilisat dann gemeinsam mit der immobilisierten Pronase verwendet, um mittels Fließ-Injektions-Analyse Aspartam-Konzentrationen zu bestimmen. Der Zellextrakt ersetzt dabei die Phenylalanin-Dehydrogenase, auf die Chymotrypsin-Säule kann hierbei verzichtet werden. Anstelle von $NAD^+$ wird bei dieser Analysenmethode $NADP^+$ eingesetzt. Mit Standardlösungen verschiedener Aspartam-Konzentrationen wurde eine Eichkurve erstellt, die in Fig. 5 gezeigt ist. Die Fig. verdeutlicht, daß das Signal am Fluoreszenz-Photometer propertional zur Aspartam-Konzentrationn ansteigt.

**Patentansprüche**

1. Verfahren zu Bestimmung des Gehalts an L-$\alpha$-Aspartyl-L-phenylalanin-methylester (Asp-PheOMe; Aspartam®) in wäßriger Lösung durch enzymatische Spaltung in Asparaginsäure und Phenylalaninmethylester mittels Peptidase und nachfolgende enzymatische Nachweisreaktion,
**dadurch gekennzeichnet,**
daß man als Nachweisreaktion eine adenindinukleotid-cokatalysierte enzymatische Reaktion herbeiführt, indem man entweder
(a) die gebildete Asparaginsäure mittels eines Asparaginsäure in Gegenwart von $NADP^+$ umsetzenden enzymhaltigen Zellextrakts unter Zugabe von $NADP^+$ umsetzt und die Konzentration des Aspartams über die NADPH- oder $NH_3$-Bildung ermittelt oder
(b) die Estergruppe vom Phenylalaninmethylester enzymatisch mittels Chymotrypsin abtrennt und das gebildete L-Phenylalanin enzymatisch mittels Phenylalanin-Dehydrogenase in Gegenwart von $NAD^+$ in Phenylpyruvat umwandelt und die Aspartam-Konzentration über die NADH- oder $NH_3$-Bildung ermittelt.

2. Verfahren nach Anspruch 1,
**dadurch gekennzeichnet,**
daß man die Enzyme in trägerfixierter Form anwendet.

3. Verfahren nach Anspruch 2,
**dadurch gekennzeichnet,**
daß man die Enzyme fixiert an feinteiligem mikroporösen Inertmaterial mit anlagerungsaktiven Gruppen, insb. an aktiviertem $SiO_2$-Träger mit Poren ≥ 500 Å verwendet.

4. Verfahren nach Anspruch 2,
**dadurch gekennzeichnet,**
daß man die trägerfixierten Enzyme in aufeinander folgenden Säulen verwendet, die kontinuierlich von Pufferlösung durchströmt werden, in die stromaufwärts Analysenlösung sowie $NADP^+$- bzw. $NAD^+$-Lösung injiziert werden.

5. Verfahren nach einem der vorangehenden Ansprüche,
**dadurch gekennzeichnet,**
daß man die Coenzym-Hydrierungsprodukte NADH bzw. NADPH physikalisch, insb. fluorimetrisch, bestimmt.

6. Verfahren nach Anspruch 1,
**dadurch gekennzeichnet,**
daß man eine Probe der zu analysierenden Lösung lediglich den der Peptidase-Spaltung folgenden enzymatischen Reaktionen mit Asparaginsäure umsetzendem enzymatischen Zellextrakt (a) oder mit Chymotrypsin und mit Phenylalanin-Dehydrogenase (b) unterwirft und den so erhaltenen Wert vom nach Anspruch 1 ermittelten Wert abzieht.

7. Enzymatischer Zellextrakt zur Durchführung des Verfahrens nach einem der Ansprüche 1-6, mit Asparaginsäure in Gegenwart von $NADP^+$ unter Bildung von NADPH und $NH_3$ umwandelnder Aktivität, gewonnen aus Zellisolaten von Kompostierungsproben.

8. Enzymhaltiger Zellextrakt nach Anspruch 7, gewonnen aus dem Stamm Asp-1 der Hinterlegungs-Nr. DSM 6705 oder aus seinen Mutanten.

9. Anordnung zur Bestimmung von Aspartam nach einem der Ansprüche 1 bis 6,
**gekennzeichnet durch**
einen kontinuierlich durchströmten Leitungszug mit aufeinander folgenden Säulen mit trägerfixierter Peptidase (E1), und (a) mit asparaginsäure-aktiven Zellextrakt (E4) oder (b) mit Chymotrypsin (E2) und Phenylalanin-Dehydrogenase (E3) jeweils mit einem (E4 oder E3) nachfolgenden Detektor (F) und vor den Säulen befindlicher Einspeisung von Probelösung (P3) sowie einer Einspeisung (P2) von $NADP^+$- bzw. $NAD^+$-Lösung vor der Zellextrakt- bzw. vor der Dehydrogenase-Säule.

10. Anordnung nach Anspruch 9 ,
**gekennzeichnet durch**
eine wahlweise Überbrückung der ersten (Peptidase)-Säule (E1).

11. Anordnung nach Anspruch 9 oder 10,
**gekennzeichnet durch**
ein Fluorimeter als NADPH- bzw. NADH-Detektor (F).

12. Anordnung nach Anspruch 9, 10 oder 11,
**gekennzeichnet durch**
eine Einspeisung sowohl für die Probelösung (P3) als auch für die $NADP^+$- bzw. die $NAD^+$-Lösung (P2) mit ggf. anschließender Mischstrecke vor den Säulen (E1-E4) bzw. (E1-E2-E3).

13. In einem Leitungszug einer FIA-Apparatur in Aufeinanderfolge zu benutzende Enzymsäulen für FIA-Technik zur Bestimmung von Aspartam nach einem der Ansprüche 1 bis 6,
**gekennzeichnet durch**

einen Analysensatz (A) von 2 Säulen mit trägerfixierter Peptidase (E1) und asparaginsäure-aktivem Zellextrakt (E4) oder (B) von 3 Säulen mit Peptidase (E1), Chymotrypsin(E2) und Phenylalanin-Dh (E3).

## Claims

1. A method of determining the content of L-α-aspartyl-L-phenylalanine methyl ester (Asp-PheOMe; aspartame®) in aqueous solution by enzymatic cleavage into aspartic acid and phenylalanine methyl ester by means of peptidase and a subsequent enzymatic detection reaction, characterised in that an enzymatic reaction co-catalysed by adenine dinucleotide is performed as the detection reaction, in that either (a) the aspartic acid formed is reacted, with the addition of $NADP^+$, by means of an enzyme-containing cell extract which converts aspartic acid in the presence of $NADP^+$ and the concentration of aspartame is determined via the formation of NADPH or $NH_3$, or
(b) the ester group of the phenylalanine methyl ester is split off enzymatically by means of chymotrypsin and the L-phenylalanine formed is converted enzymatically into phenyl pyruvate by means of phenylalanine dehydrogenase in the presence of $NAD^+$ and the aspartame concentration is determined via the formation of NADH or $NH_3$.

2. A method according to claim 1, characterised in that the enzymes are used in a form fixed on a support.

3. A method according to claim 2, characterised in that the enzymes are fixed on finely-divided microporous inert material with addition reaction-active groups, particularly on activated $SiO_2$ supports with pores ≳ 500 Å.

4. A method according to claim 2, characterised in that the enzymes fixed on a support are used in successive columns through which buffer solution flows continuously and into which the analysis solution and $NADP^+$ or $NAD^+$ solution are injected upstream.

5. A method according to any one of the preceding claims, characterised in that the coenzyme hydrogenation products NADH or NADPH are determined physically, particularly fluorometrically.

6. A method according to claim 1, characterised in that a sample of the solution to be analysed is subjected only to the enzymatic reactions which follow the peptidase cleavage, with an enzymatic cell extract which reacts with aspartic acid (a) or with chymotrypsin and with phenylalanine dehydrogenase (b), and the value thus obtained is subtracted from the value determined according to claim 1.

7. An enzymatic cell extract for carrying out the method according to any one of claims 1-6, with an activity which converts aspartic acid in the presence of $NADP^+$ with the formation of NADPH and $NH_3$, obtained from cell isolates from compost preparation samples.

8. An enzyme-containing cell extract according to claim 7, obtained from the Asp-1 strain of deposition number DSM 6705 or its mutants.

9. An arrangement for determining aspartame according to any one of claims 1 to 6, characterised by a continuous-flow line section with successive columns containing peptidase (E1) fixed on a support, and (a) containing aspartic acid-active cell extract (E4) or (b) containing chymotrypsin (E2) and phenylalanine dehydrogenase (E3) having in each case a subsequent (to E4 or E3) detector (F) and a supply of sample solution (P3) situated upstream of the columns and having a supply (P2) of $NADP^+$ or $NAD^+$ solution upstream of the cell extract- or dehydrogenase column, respectively.

10. An arrangement according to claim 9, characterised by optional by-passing of the first (peptidase) column (E1).

11. An arrangement according to claim 9 or 10, characterised by a fluorometer as the NADPH or NADH detector (F).

12. An arrangement according to claim 9, 10 or 11, characterised by a supply both for the sample solution (P3) and for the $NADP^+$ or $NAD^+$ solution (P2), optionally with a subsequent mixing section upstream of

the columns (E1-E4) or (E1-E2-E3), respectively.

13. Enzyme columns for use in succession in a line section of an FIA (flow injection analysis) apparatus for the FIA technique for the determination of aspartame according to any one of claims 1 to 6, characterised by an analysis set (A) of two columns with peptidase (E1) fixed on a support and aspartic acid-active cell extract (E4), or (B) of three columns with peptidase (E1), chymotrypsin (E2) and phenylalanine-Dh (E3).

## Revendications

1. Procédé pour la détermination de la teneur en ester méthylique de L-α-aspartyl-L-phénylalanine (Asp-PheOMe ; Aspartame$^{(R)}$) en solution aqueuse, par clivage enzymatique en acide aspartique et ester méthylique de phénylalanine, au moyen d'une peptidase et d'une réaction enzymatique ultérieure de détection, caractérisé en ce qu'on effectue, comme réaction de détection, une réaction enzymatique co-catalysée avec un adénine-dinucléotide,
   (a) soit en faisant réagir l'acide aspartique formé, au moyen d'un extrait cellulaire contenant une enzyme et faisant réagir l'acide aspartique en présence de $NADP^+$, avec addition de $NADP^+$, et en déterminant la concentration d'aspartame en fonction de la formation de NADPH ou de $NH_3$,
   (b) soit en séparant le groupe ester de l'ester méthylique de phénylalanine, par voie enzymatique à l'aide de chymotrypsine et transformant la L-phénylalanine formée en pyruvate de phényle, par voie enzymatique à l'aide de phénylalanine-déshydrogénase en présence de $NAD^+$, et en déterminant la concentration d'aspartame en fonction de la formation de NADH ou de $NH_3$.

2. Procédé selon la revendication 1, caractérisé en ce qu'on utilise les enzymes sous une forme fixée sur un support.

3. Procédé selon la revendication 2, caractérisé en ce qu'on utilise les enzymes fixées sur un matériau inerte microporeux finement divisé avec des groupes actifs par addition, en particulier sur un support de $SiO_2$ activé, avec des pores $\gtrsim$ 500 Å.

4. Procédé selon la revendication 2, caractérisé en ce qu'on utilise les enzymes fixées sur le support dans des colonnes successives, qui sont traversées en continu par une solution tamponnée, dans lesquelles on injecte, en amont, la solution à analyser, ainsi qu'une solution de $NADP^+$ ou une solution de $NAD^+$.

5. Procédé selon l'une des revendications précédentes, caractérisé en ce qu'on détermine les produits d'hydrogénation de la coenzyme, c'est-à-dire NADH ou NADPH, au moyen d'une analyse physique, notamment fluorométrique.

6. Procédé selon la revendication 1, caractérisé en ce qu'on soumet un échantillon de la solution à analyser seulement aux réactions enzymatiques suivant le clivage par la peptidase, avec l'extrait cellulaire enzymatique (a) faisant réagir l'acide aspartique ou avec la chymotrypsine et avec la phénylalanine-déshydrogénase (b), et on soustrait la valeur ainsi obtenue de la valeur déterminée selon la revendication 1.

7. Extrait cellulaire enzymatique pour la mise en oeuvre du procédé selon l'une des revendications 1 à 6, obtenu à partir de produits cellulaires isolés dans des échantillons de compostage et ayant une activité de transformation de l'acide aspartique, en présence de $NADP^+$, avec la formation de NADPH et de $NH_3$.

8. Extrait cellulaire enzymatique selon la revendication 7, obtenu à partir de la souche Asp-1 déposée sous le numéro DSM 6705 ou à partir de ses mutants.

9. Agencement pour la détermination de l'aspartame selon l'une des revendications 1 à 6, caractérisé en ce qu'il comporte un ensemble de canalisations traversées en continu, avec des colonnes successives avec une peptidase (E1) fixée sur un support, et (a) avec un extrait cellulaire (E4) actif avec l'acide aspartique ou (b) avec de la chymotrypsine (E2) et de la phénylalanine-déshydrogénase (E3), suivies dans chaque cas d'un détecteur (F), et d un dispositif d'injection de solution d'échantillon (P3) situé a l'avant des colonnes, ainsi qu'un système d'injection (P2) de solution de $NADP^+$ ou de $NAD^+$ à l'avant

de la colonne d'extrait cellulaire ou à l'avant de la colonne de déshydrogénase.

**10.** Agencement selon la revendication 9, caractérisé par un rattachement au choix de la première colonne (E1) (peptidase).

**11.** Agencement selon la revendication 9 ou 10, caractérisé en ce qu'il comporte un fluoromètre comme détecteur (F) de NADPH ou de NADH.

**12.** Agencement selon la revendication 9, 10 ou 11, caractérisé en ce qu'il comporte un système d'injection pour la solution d'échantillon (P3) ainsi que pour la solution de $NADP^+$ ou la solution de $NAD^+$ (P2), suivi éventuellement d'une section de mélange à l'avant des colonnes (E1-E4) ou (E1-E2-E3).

**13.** Colonnes enzymatiques à utiliser successivement dans un jeu de canalisations d'un appareil de FIA pour la technique FIA de détermination de l'aspartame selon l'une des revendications 1 à 6, caractérisées par un ensemble analytique (A) formé de 2 colonnes avec une peptidase (E1) fixée sur un support et un extrait cellulaire (E4) actif avec l'acide aspartique, ou un ensemble analytique (B) formé de 3 colonnes avec une peptidase (E1), de la chymotrypsine (E2) et de la phénylalanine-déshydrogénase (E3).

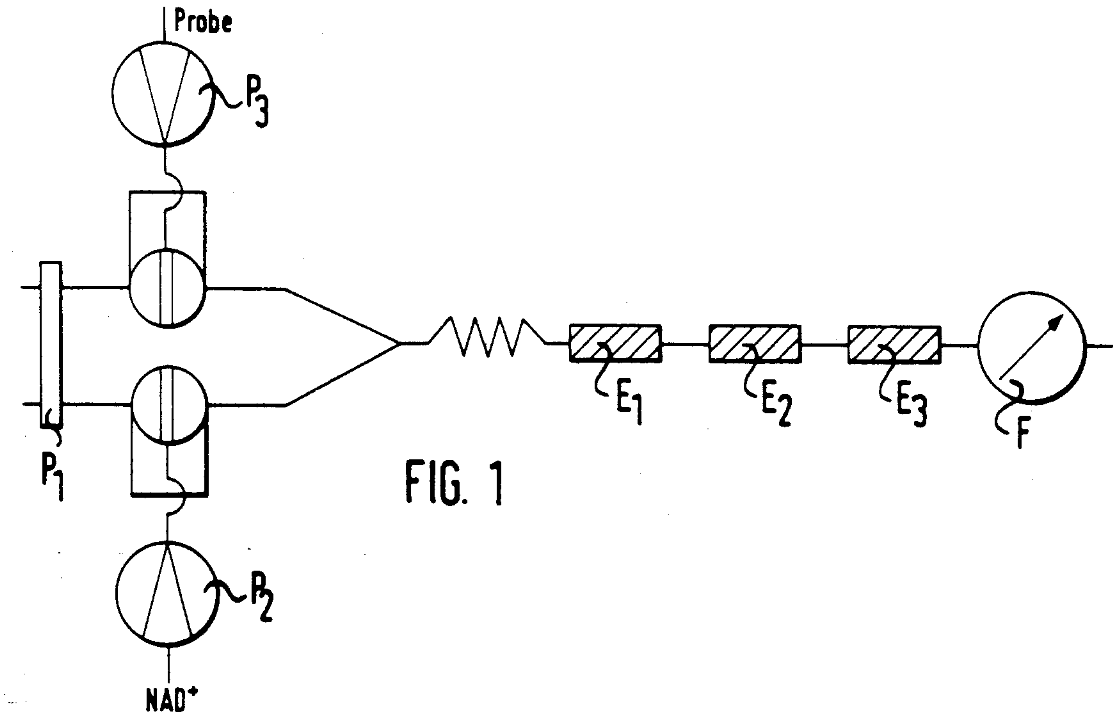


FIG. 1

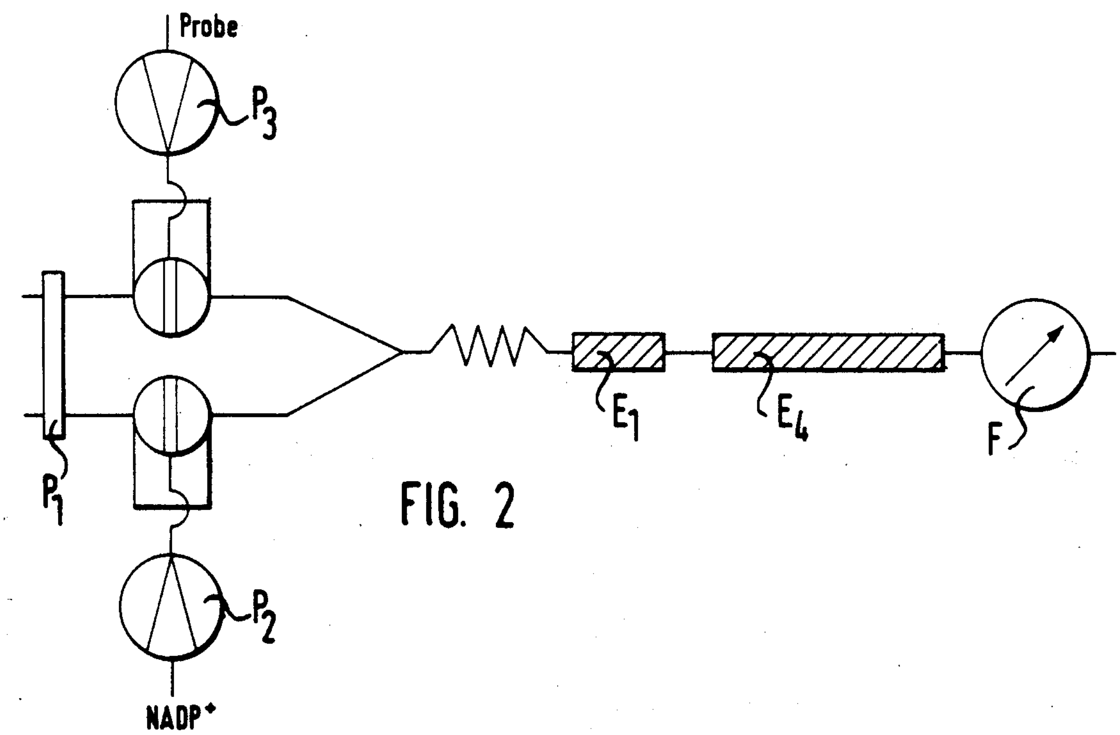


FIG. 2

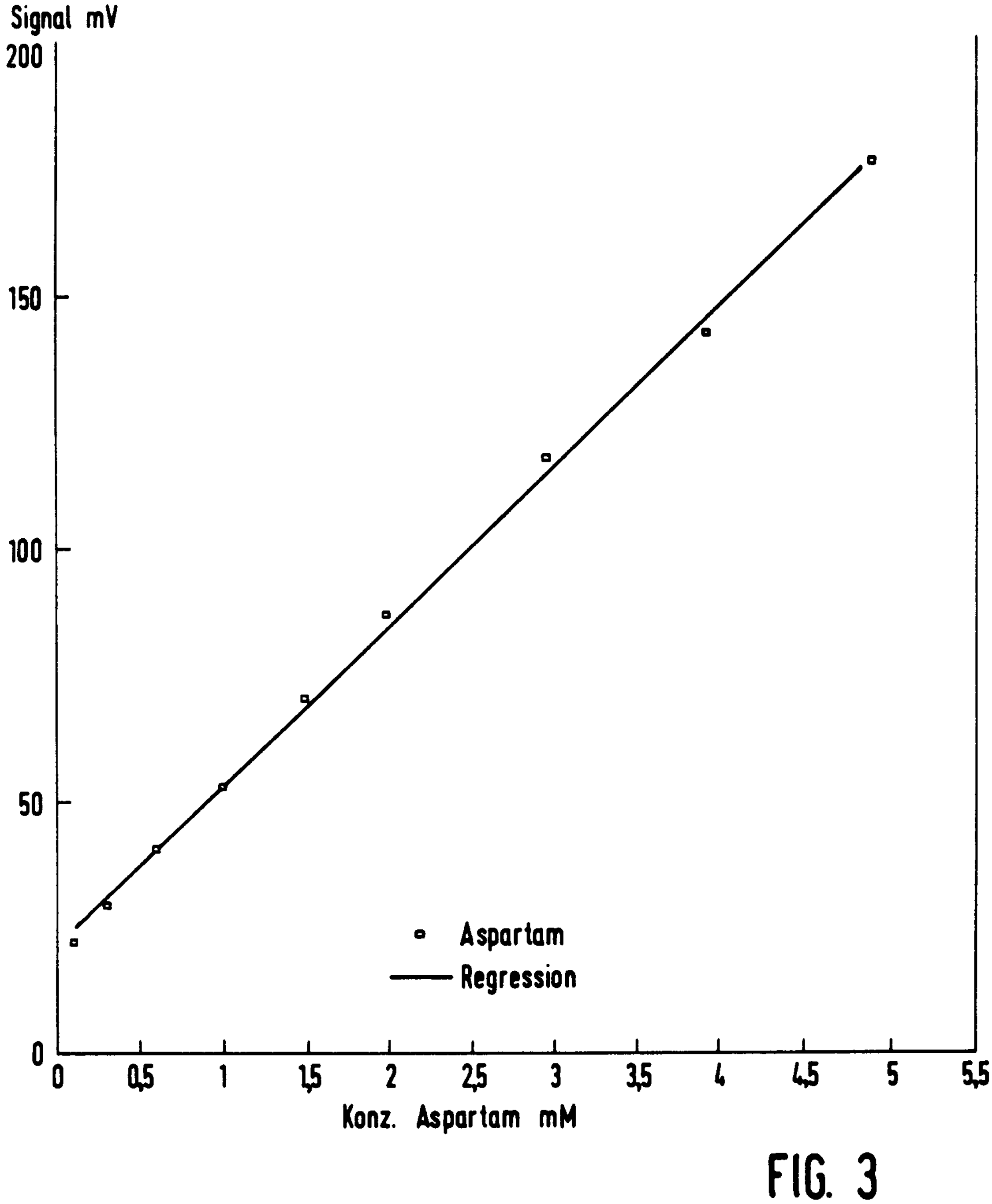
Signal mV
200
150
100
50
0
0
0,5
1
1,5
2
2,5
3
3,5
4
4,5
5
5,5
Konz. Aspartam mM
Aspartam
Regression

FIG. 3

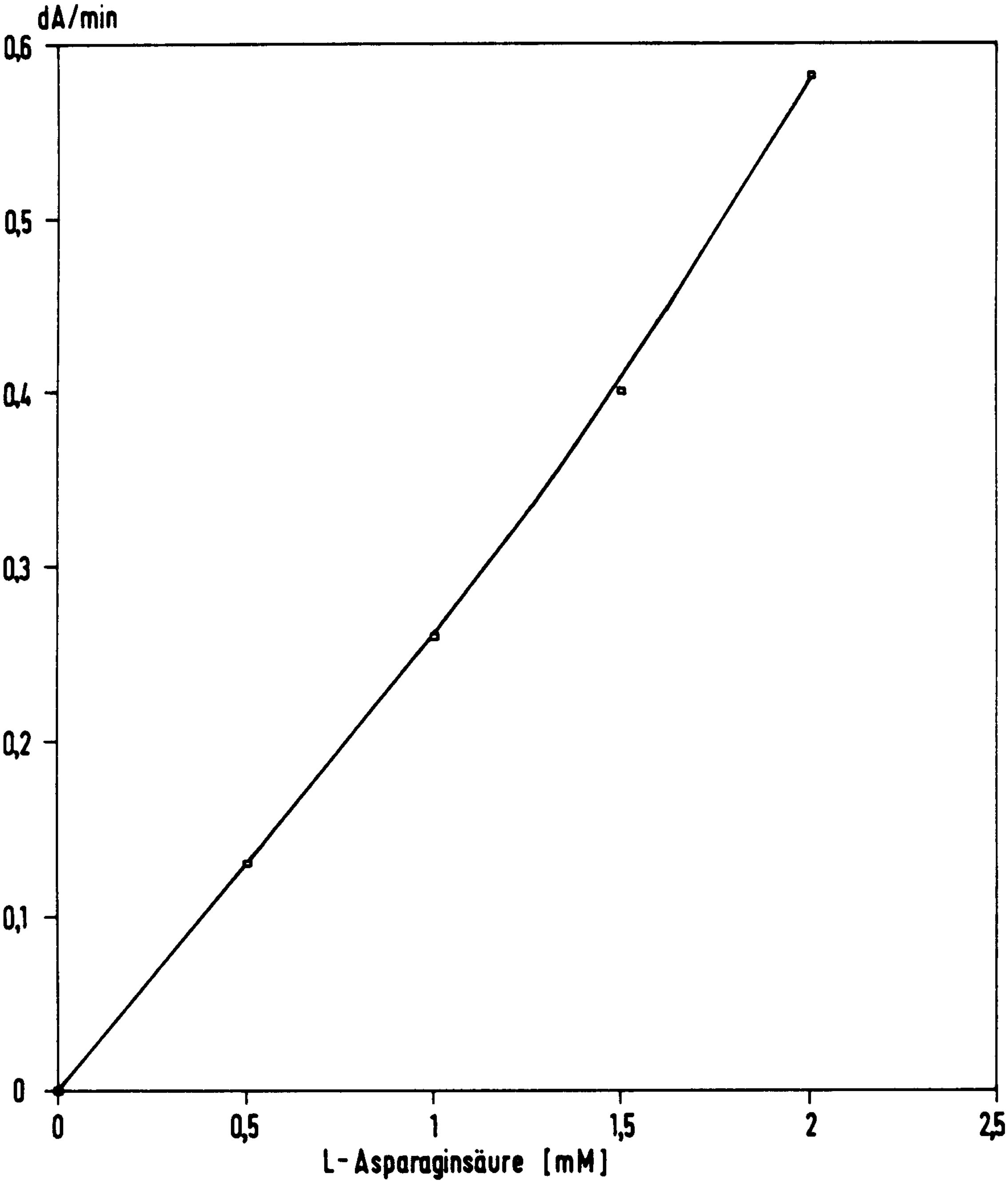


FIG. 4

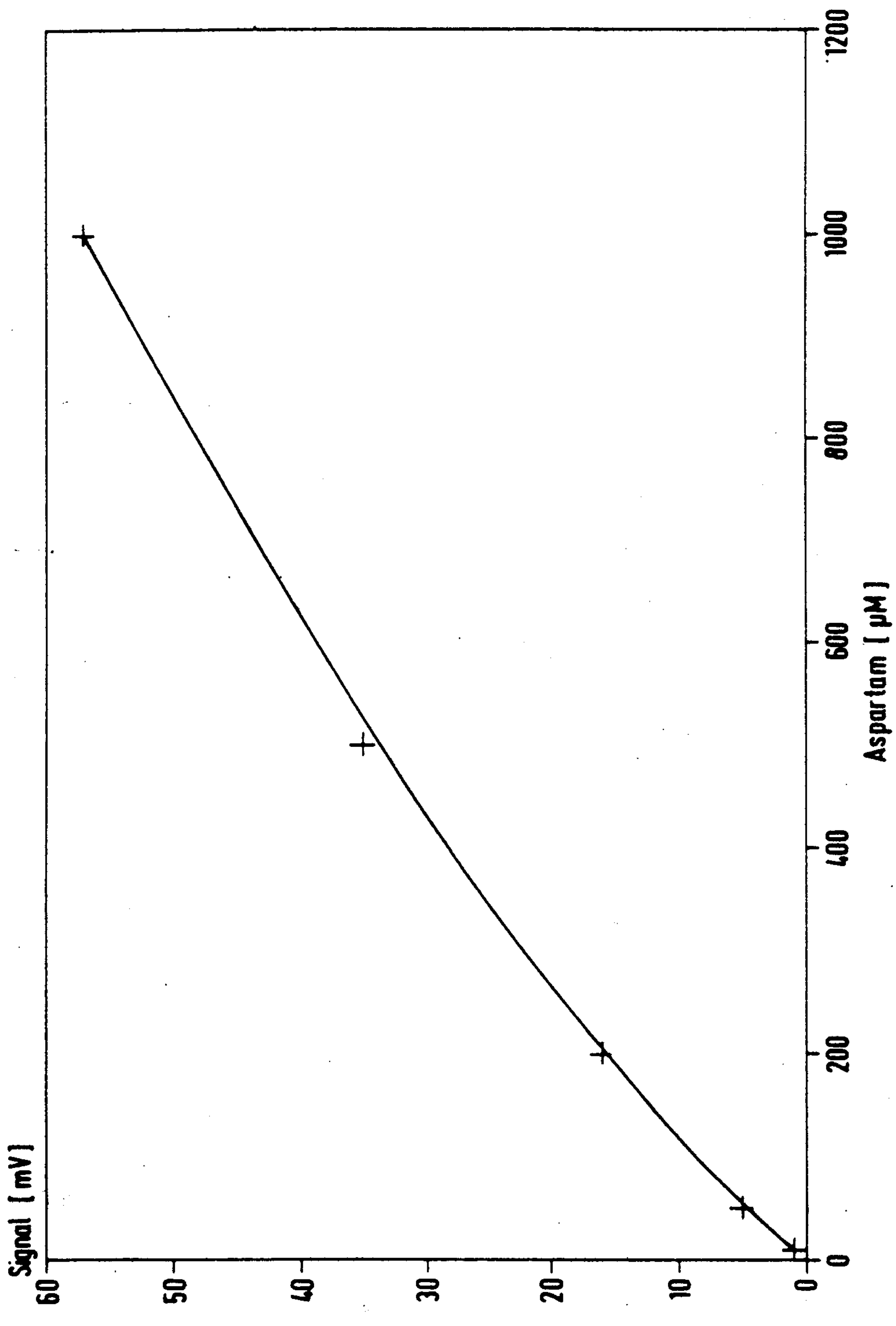
Signal [mV]
60
50
40
30
20
10
0
200
400
600
800
1000
1200
Aspartam [µM]

FIG. 5